# EUROPEAN PATENT SPECIFICATION

(11) **EP 0 995 409 B1**
(45) Date of publication and mention of the grant of the patent: **12.03.2008**
(21) Application number: 99116209.0
(22) Date of filing: 17.08.1999
(51) Int. Cl.: A61F 2/08

(54) **A device for anchoring a crucial ligament transplant into the knee joint parts**
Vorrichtung zum Befestigen eines kritischen Ligamenttranspants in Kniegelenkteile
Dispositif de fixation d'un ligament critique dans des parties d'articulation du genou

(30) Priority: 26.08.1998 DE 29815290 U
(43) Date of publication of application: 26.04.2000
(73) Proprietor: Howmedica GmbH, D-24232 Schönkirchen (DE)
(72) Inventor: Vécsei, Vilmos, Prof. Dr. med., 1140 Vienna (AT); Seitz, Helmut, Prof. Dr., 8665 Langenwang (AT); Harder, Hans Erich, 24253 Probsteierhagen (DE)
(74) Representative: Kopf, Korbinian Paul

(56) References cited:
- EP-A- 0 340 159
- FR-A- 2 671 717
- FR-A- 2 696 925
- FR-A- 2 740 324
- US-A- 5 720 753

## Description

The invention relates to a device for anchoring a crucial ligament transplant into the knee joint parts, according to claim 1.

It is an established method of knee ligament surgery for a torn crucial ligament, to apply a patella tendon bone transplant. In the first post-operative weeks the intensity of the early functional rehabilitation is limited essentially by the breaking load of the transplant anchoring. It is known to carry out the anchoring with a so-called interference screw. With this the end of the tendon or the bone block connected to the tendon is fixed in a bore of the appropriate knee joint part with the help of a screw. It however has been shown that with this type of fastening problems and complications occur. Thus amongst other things damage to the bone block, uncontrollable displacements of the bone block, fracture of the bone block as well as damage of the ligament transplant take place. Also an erroneous positioning of the interference screw has been observed.

FR-A-2 671 717 discloses an anchor system suitable for anchoring a crucial ligament transplant. The anchor system comprises a generally cylindrical sleeve of metallic, bio-compatible material, with at least two axis-parallel slots. The system further comprises a screw 2. When inserting the screw into the sleeve the slotted sections of the sleeve spread apart such that the anchor system can be anchored in bone tissue. The disclosed anchor system has the disadvantage that due to a roughening being provided at almost the full periphery of the sleeve the mechanical strength of the sleeve is weakened.

It is therefore the object of the invention to provide a device for anchoring a crucial ligament transplant, which does not have the above disadvantage, can be simply applied and ensures an effective anchoring.

This object is achieved by the features of claim 1.

The device according to the invention consists of two parts, specifically a sleeve and a screw. The screw comprises a threaded shank as well as a head on the end of the shank. The sleeve and screw consist of body-compatible material, wherein the sleeve is preferably manufactured of titanium.

The sleeve comprises a head at one end and at least two axis-parallel slots which are distanced by a circumferential distance and which extend from the other end of the sleeve in the direction of the head. The sleeve comprises further an inner threaded section into which the screw shank is screwed. Since the thread-free sleeve bore section which lies towards the free end of the sleeve in diameter is equal or smaller than the diameter of the inner thread, there is effected a spreading apart of the sleeve at the free end when the screw shank enters into the thread-free part of the sleeve.

The outer side of the sleeve between the slots is provided with a roughening. With the help of such a device therefore a bone block or the patella tendon itself may be effectively fastened against the walling of a bore which previously has been brought into the joint parts from the outside.

According to one formation of the invention between the head and the roughening there is provided a smoother outer section for the sleeve. The roughening leads as known to a weakening of the strength of the sleeve. A smooth outer side on the other hand has a higher strength so that the strength of the sleeve as a whole is very high. The slots may extend into the smooth section or end at these.

According to one formation of the invention the roughening is formed by annular ribs concentric to the axis of the sleeve. In cross section the ribs may be formed such that there results a sawtooth-like course, which effects a particularly effective anchoring of the transplant in the bore.

Preferably there are provided four slots arranged at a circumferential distance of 90°.

According to a further formation of the invention on the outer side of the head of the sleeve there is attached a marking which has a fixed relation to the slots. By way of this the surgeon may ensure that a spread out sector of the sleeve effectively comes into engagement with the transplant. It is avoided that a slot region directly engages with the transplant, which would reduce the effectiveness of the anchoring.

The head of the sleeve is preferably a polygon as also the head of the screw. The polygon head of the sleeve serves to hold this against rotation when the screw is screwed via its head into the sleeve. It is to be understood that the diameter of the sleeve or the width of the head of the screw is considerably smaller than that of the head of the sleeve.

According to a further formation of the invention the length of the sleeve is between 26 and 30 mm, preferably 28 mm. The diameter of the sleeve varies between 4 and 6 mm, preferably is 5 mm, measured in the smooth region near to the head or in the tip diameter of the roughening.

The invention is hereinafter described in more detail by way of an embodiment example shown in the drawings.
- Fig. 1: shows two devices according to the invention after their implantation in the knee.
- Fig. 2: shows the lateral view of a device according to the invention.
- Fig. 3: shows an end view of the device according to Fig. 2 in the direction of arrow 3.
- Fig. 4: shows a longitudinal section through the sleeve of the device according to Fig. 2.
- Fig. 5: shows the lateral view of a screw of the device according to Fig. 2.

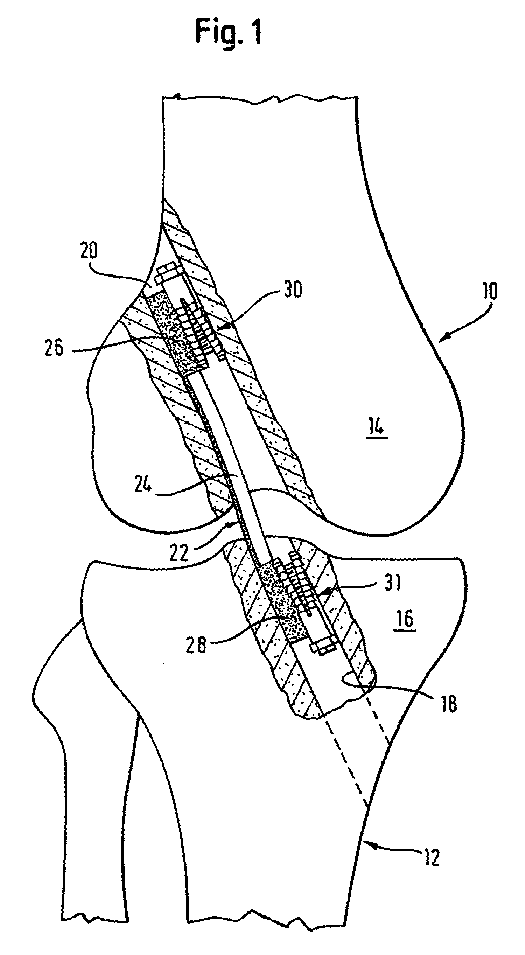

In Fig. 1 there is indicated a femur 10 and a tibia 12 with the corresponding joint parts 14 and 16 respectively. One recognizes a bore 18 in the joint part 16 and a bore 20 in the joint part 14. They extend outwardly from the femoral and tibial reference point of a somewhat front crucial ligament on the joint part 14 and the joint part 16 respectively. The bores 18, 20 which in each case are carried out from the outside to the inside with the operation, are aligned to one another when the joints 14, 16 face one another in their position shown in Fig. 1 (stretched knee). Also the outer reference point for the drilling tool on creating the bores 18, 20 is directed according to this.

In the bores 18, 20 there is located a patella tendon transplant 22, consisting of a tendon section 24 and two bone blocks 26, 28 at the ends of the tendon section 24. The transplant 22 which during the operation is previously removed, is applied into the bores 18, 20 in the manner and way shown in Fig. 1. The fastening of the bone blocks 26, 28 is effected with fastening devices 30 and 32 respectively, which are yet described further below. In this manner the transplant 22 is fastened in the joint parts 14, 16 and may as a result assume the function of a crucial ligament.

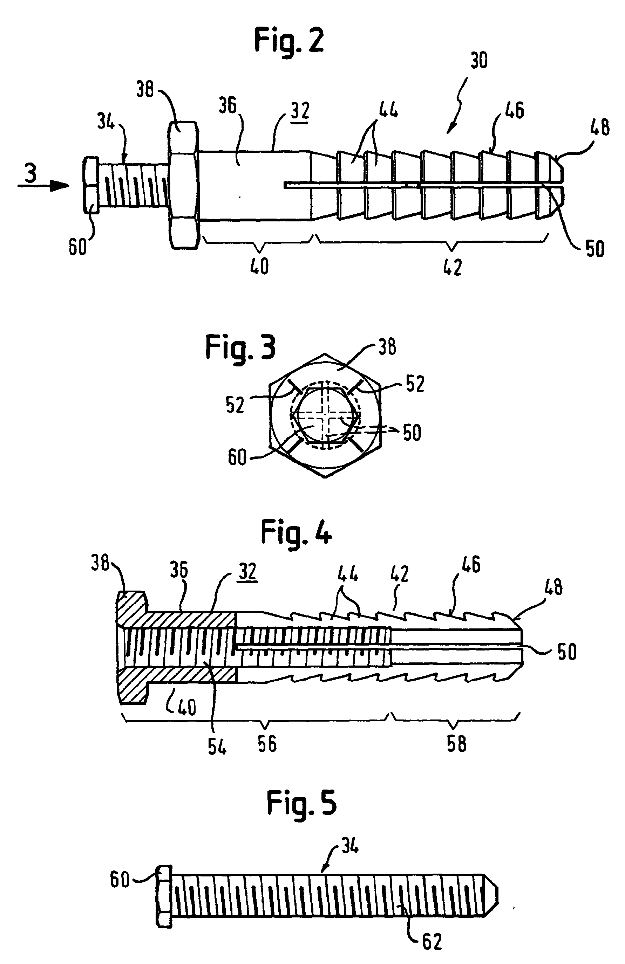

A device according to Fig. 1, for example the device 30, is shown in more detail in the Figures 2 to 5. It is composed of a sleeve 32 of titanium and a screw 34 of a suitable body-compatible material or a suitable metal alloy. The sleeve 32 has a shank 36 and a head 38 at one end. The shank 36 comprises a smooth section 40 which is directly neighbouring the head 38 and which blends into a ribbed section 42. The ribs 44 of the ribbed section 42 are concentric to the longitudinal axis of the shank 36. They are shaped by oblique annular grooves 46 in a manner such that in section there results a sawtooth-like profile. At the end the shank 36 comprises a conical introduction section 48.

Four axis-parallel slots arranged at a circumferential distance of 90°, of which one is recognized at 50, extend from the introduction section 48 up to in the smooth section 40 and subdivide this section in four sectors.

As can be recognized from Fig. 3 the head 38 is a hexagonal head whose width is significantly greater than the diameter of the smooth section 40. As can further be recognized from Fig. 3 there are provided four line markings 52 on the head 38, which are arranged at a distance of 90°. The markings lie in each case centrally between neighbouring slots 50. The surgeon who handles the device according to Fig. 2 knows as a result where the sectors in the rotational direction lie in each case.

As is deduced from Fig. 4, the sleeve 32 and the shank 36 comprises a bore 54 which completely passes through. An inner threaded section 56 extends from the head 38 over approximately two thirds of the shank 36 and then blends into a section 58 with a diameter which corresponds roughly to the tip diameter of the thread.

In Fig. 5 the screw 34 is represented which comprises a hexagonal head 60 and a threaded shank 62. The threaded shank 62 is shorter than the sleeve shank 36. The head 60 is significantly smaller than the head 38 of the sleeve 32.

If the screw 34 is screwed into the sleeve 32, the sector sections in the ribbed section 42 spread radially outwards. This condition is shown in Fig. 1. In this manner the bone blocks 26, 28 are effectively pressed by the sectors against the walling of the bore. It is therefore possible to already prematurely load the transplant, without there existing the danger that the anchoring yields.

With the treatment firstly after a suitable preparation the bores 18, 20 are shaped into which then the transplant 22 is placed. The diameter of the bore 18, 20 is significantly greater than the thickness of the bone block 26, 28. After positioning the transplant 22 and its temporary fastening with the help of means which here are not shown, from the outside firstly one of the two devices 30, 32 are introduced and actuated with the help of screw tools. The head 38 of the device 30 with a tool is held against rotation so that the screw 34 on the head can be screwed into the sleeve 32 with a simultaneous spreading of the ribbed section 42 for the purpose of pressing the bone blocks 26 or 28 onto the bore wall. If the one bone block 26, 28 is anchored there is then effected the anchoring of the other bone block in the same way.

## Claims

1. A device for anchoring a crucial ligament transplant in the knee joint parts, comprising
• a generally cylindrical sleeve (30) of metallic, bio-compatible material, with at least two axis-parallel slots (50) which are distanced in the circumferential direction and which extend from one end over a larger length of the sleeve (30), with a roughening (42) of a sleeve section in the region of the slots (50), with a head (38) at the other end of the sleeve (30) and with an inner threaded section (56) which extends from the head (38) up to into the region of the slots (50), wherein the connecting section (58) of the sleeve bore (54) has a diameter which is equal or smaller than the tip diameter of the inner threaded section (56), and comprising
• a screw (34) with a threaded shank (62) which is screwable into the inner thread (54) and with a head (60) at one end of the shank (62), wherein the length of the shank (62) is smaller than that of the sleeve (30), **characterized in that**
the sleeve (30) between the head (38) and the roughening (42) comprises a smooth outer section (40) and
the slots (50) extend into the smooth section (40).

2. A device according to claim 1, **characterized in that**
the sleeve (30) is formed of titanium.

3. A device according to claim 1 or 2, **characterized in that**
the roughening is formed by annular ribs (44) concentric to the axis of the sleeve (30).

4. A device according to claim 3, **characterized in that**
the annular ribs are shaped such that in section there results a sawtooth-like course.

5. A device according to claims 1 to 4, **characterized in that**
there are provided four slots (50) at a circumferential distance of 90°.

6. A device according to one of claims 1 to 5, **characterized in that**
the head (38, 60) of the sleeve (30) and/or screw (34) is a polygonal head.

7. A device according to one of the claims 1 to 6, **characterized in that**
the diameter of the screw head (60) is smaller than that of the sleeve head (38).

8. A device according to one of claims 1 to 7, **characterized in that**
the sleeve (30) has a length of 26 to 30 mm, preferably of about 28 mm.

9. A device according to one of the claims 1 to 8, **characterized in that**
the sleeve has a diameter of 4 to 6 mm, preferably of 5 mm.

10. A device according to one of claims 1 to 9, **characterized in that**
on the outer side of the head (38) there is provided a marking (52) which has a fixed relation to the position of the slot (50).

## Patentansprüche

1. Vorrichtung zum Verankern eines Kreuzbandtransplantats in den Kniegelenksteilen, aufweisend
- eine im Allgemeinen zylindrische Hülse (30) aus metallischem, biokompatiblem Material mit mindestens zwei achsenparallelen Schlitzen (50), welche in der Umfangsrichtung voneinander entfernt sind und welche sich von einem Ende aus über eine größere Länge der Hülse (30) erstrecken, mit einer Aufrauung (42) eines Hülsenabschnittes im Bereich der Schlitze (50), mit einem Kopf (38) am anderen Ende der Hülse (30) und mit einem Innengewindeabschnitt (56), welcher sich vom Kopf (38) bis in den Bereich der Schlitze (50) erstreckt, wobei der Verbindungsabschnitt (58) der Hülsenbohrung (54) einen Durchmesser aufweist, der gleich dem oder geringer als der Spitzendurchmesser des Innengewindeabschnittes (56) ist, und aufweisend
- eine Schraube (34) mit einem Gewindeschaft (62), der in das Innengewinde (54) schraubbar ist, und mit einem Kopf (60) an einem Ende des Schaftes (62), wobei die Länge des Schaftes (62) kürzer als die der Hülse (30) ist, **dadurch gekennzeichnet, dass**
die Hülse (30) zwischen dem Kopf (38) und der Aufrauung (42) einen glatten Außenabschnitt (40) aufweist und
sich die Schlitze (50) in den glatten Abschnitt (40) erstrecken.

2. Vorrichtung nach Anspruch 1, **dadurch gekennzeichnet, dass**
die Hülse (30) aus Titan geformt ist.

3. Vorrichtung nach Anspruch 1 oder 2, **dadurch gekennzeichnet, dass**
die Aufrauung durch ringförmige Rippen (44) gebildet ist, die konzentrisch zur Achse der Hülse (30) verlaufen.

4. Vorrichtung nach Anspruch 3, **dadurch gekennzeichnet, dass**
die ringförmigen Rippen derart geformt sind, dass sich im Schnitt ein sägezahnähnlicher Verlauf ergibt.

5. Vorrichtung nach den Ansprüchen 1 bis 4, **dadurch gekennzeichnet, dass**
vier Schlitze (50) mit einem Umfangsabstand von 90 ° bereitgestellt sind.

6. Vorrichtung nach einem der Ansprüche 1 bis 5, **dadurch gekennzeichnet, dass**
der Kopf (38, 60) der Hülse (30) und/oder der Schraube (34) ein vieleckiger Kopf ist.

7. Vorrichtung nach einem der Ansprüche 1 bis 6, **dadurch gekennzeichnet, dass**
der Durchmesser des Schraubenkopfes (60) kleiner ist als der des Hülsenkopfes (38).

8. Vorrichtung nach einem der Ansprüche 1 bis 7, **dadurch gekennzeichnet, dass**
die Hülse (30) eine Länge von 26 bis 30 mm, vorzugsweise von etwa 28 mm, aufweist.

9. Vorrichtung nach einem der Ansprüche 1 bis 8, **dadurch gekennzeichnet, dass**
die Hülse einen Durchmesser von 4 bis 6 mm, vorzugsweise 5 mm, aufweist.

10. Vorrichtung nach einem der Ansprüche 1 bis 9, **dadurch gekennzeichnet, dass**
auf der Außenseite des Kopfes (38) eine Markierung (52) bereitgestellt ist, welche eine feste Beziehung zu der Position des Schlitzes (50) aufweist

## Revendications

1. Dispositif pour ancrage d'un greffon de ligament croisé dans les parties d'articulation du genou, comportant
• un manchon sensiblement cylindrique (30) en un matériau biocompatible métallique, ayant au moins deux fentes axiales parallèles (50) qui sont séparées dans la direction circonférentielle et qui s'étendent à partir d'une première extrémité au-dessus d'une plus grande longueur du manchon (30), avec une rugosité (42) d'un tronçon de manchon dans la zone des fentes (50), avec une tête (38) à l'autre extrémité du manchon (30) et avec un tronçon fileté intérieur (56) qui s'étend à partir de la tête (38) jusque dans la zone des fentes (50), la partie de connexion (58) de l'alésage de manchon (54) ayant un diamètre qui est égal ou inférieur au diamètre de pointe du tronçon fileté intérieur (56), et comportant
• une vis (34) ayant une tige filetée (62) qui peut être vissée dans le filet intérieur (54), et avec une tête (60) à une première extrémité de la tige (62), la longueur de la tige (62) étant plus petite que celle du manchon (30), **caractérisé en ce que**
le manchon (30), entre la tête (38) et la rugosité (42), comporte un tronçon extérieur lisse (40) et
les fentes (50) s'étendent dans le tronçon lisse (40).

2. Dispositif selon la revendication 1, **caractérisé en ce que**
le manchon (30) est réalisé en titane.

3. Dispositif selon la revendication 1 ou 2, **caractérisé en ce que**
la rugosité est formée par des nervures annulaires (44) concentriques par rapport à l'axe du manchon (30).

4. Dispositif selon la revendication 3, **caractérisé en ce que**
les nervures annulaires sont mises en forme de sorte qu'en coupe, elles ont en résultat une trajectoire analogue à des dents de scie.

5. Dispositif selon l'une quelconque des revendications 1 à 4, **caractérisé en ce que**
quatre fentes (50) sont agencées à une distance circonférentielle de 90°.

6. Dispositif selon l'une quelconque des revendications 1 à 5, **caractérisé en ce que**
la tête (38, 60) du manchon (30) et/ou de la vis (34) est une tête polygonale.

7. Dispositif selon l'une quelconque des revendications 1 à 6, **caractérisé en ce que**
le diamètre de la tête de vis (60) est plus petit que celui de la tête de manchon (38).

8. Dispositif selon l'une quelconque des revendications 1 à 7, **caractérisé en ce que**
le manchon (30) a une longueur de 26 à 30 mm, de préférence d'environ 28 mm.

9. Dispositif selon l'une quelconque des revendications 1 à 8, **caractérisé en ce que**
le manchon a un diamètre de 4 à 6 mm, de préférence de 5 mm.

10. Dispositif selon l'une quelconque des revendications 1 à 9, **caractérisé en ce que**
sur le côté extérieur de la tête (38) est agencé un marquage (52) qui a une relation fixe par rapport à la position de la fente (50).
